# EUROPÄISCHE PATENTSCHRIFT

(11) **EP 3 007 739 B1**
(45) Veröffentlichungstag und Bekanntmachung des Hinweises auf die Patenterteilung: **14.04.2021**
(21) Anmeldenummer: 14744765.0
(22) Anmeldetag: 16.06.2014
(51) Int. Cl.: A61L 27/44, A61L 27/48

(54) **HERSTELLUNG VON HALBZEUGEN FÜR IMPLANTATE AUF BASIS VON KUNSTSTOFF**
PRODUCTION OF SEMIFINISHED GOODS FOR IMPLANTS BASED ON PLASTIC
FABRICATION DE PRODUITS SEMI-FINIS POUR IMPLANTS À BASE DE MATIÈRE PLASTIQUE

(30) Priorität: 14.06.2013 DE 102013211175
(43) Veröffentlichungstag der Anmeldung: 20.04.2016
(73) Patentinhaber: R+K CAD CAM Technologie GmbH & Co. KG, 12681 Berlin (DE)
(72) Erfinder: ELLMANN, Daniel, 15370 Fredersdorf-Vogelsdorf (DE); KLAR, Andreas, 12681 Berlin (DE); MÜLLER, Wolf-Dieter, 12524 Berlin (DE); SCHWITALLA, Andreas, 10435 Berlin (DE); LACKMANN, Justus, 38446 Wolfsburg (DE); SPINTIG, Tobias, 13187 Berlin (DE); ABOU-EMARA, Mohamed, 13353 Berlin (DE); KALLAGE, Ilona, 10629 Berlin (DE)
(74) Vertreter: Eisenführ Speiser
(86) Internationale Anmeldenummer: PCT/EP2014/001629
(87) Internationale Veröffentlichungsnummer: WO 2014/198421

(56) Entgegenhaltungen:
- FR-A1- 2 915 088
- US-A1- 2007 111 165
- US-A1- 2009 222 091
- US-B1- 6 193 516
- Wikipedia-Auszug: Fertigungsverfahren, 22.06.2018, 4 Seiten.
- Wikipedia-Auszug: Fügen (Fertigungstechnik), 07.05.2018, 3 Seiten.
- Wikipedia-Auszug: Schweissen, 18.06.2018, 20 Seiten.
- Wikipedia-Auszug: Beschichten, 29.07.2018, 4 Seiten.
- Wikipedia-Auszug: Auftragschweissen, 04.05.2018, 2 Seiten.

## Beschreibung

Die Erfindung betrifft ein Implantat-Halbzeug aus Kunststoff, dessen Herstellung sowie die Verwendung des Implantat-Halbzeugs als Implantat oder zur Fertigung eines Implantats daraus, insbesondere die Verwendung als Dentalimplantat oder zur Fertigung eines Dentalimplantats.

Ein Implantat ist ein im Körper eingepflanztes künstliches Material, das permanent oder zumindest für einen längeren Zeitraum dort verbleiben soll. In der Humanmedizin kommen Implantate zum Einsatz, wenn ein natürliches Gewebe oder natürliches Material, wie z.B. Zähne, Knochen, Gelenke usw., seine Funktion aufgrund Erkrankung, Verschleiß oder traumatischer Schädigung nicht mehr erfüllen kann.

Im Bereich der Zahnmedizin werden sogenannte Dentalimplantate in den Kieferknochen eingesetzt, welche die Funktion einer künstlichen Zahnwurzel übernehmen. Dieses System besteht in der Regel aus zwei Teilen: Dem Implantat, welches im Kieferknochen steckt und einem Aufbau, dem sogenannten Abutment, welches den künstlichen Zahnersatz mit dem Implantat verbindet.

Zur Herstellung von Implantaten, die im menschlichen Körper allgemein eingesetzt werden, werden heutzutage verschiedene Materialien aus den Gruppen der Metalle, Keramiken und Kunststoffe herangezogen. Allen gemeinsam ist die Zulassung zur Langzeitimplantation aufgrund ihrer nachgewiesenen Biokompatibilität.

Implantate, die in knöchernen Strukturen des menschlichen Skelettes verankert werden, müssen aufgrund der hohen mechanischen Anforderungen entsprechend hohe Elastizitätsmodule aufweisen und entsprechend stabil sein. Bisher kamen deshalb hier vornehmlich Titan und Titanlegierungen, aber auch Keramiken zur Anwendung.

Titan, aber insbesondere Zirkonoxid, weisen eine hohe Festigkeit und nur eine sehr geringe physikalische Elastizität auf. Die E-Module von Titan und Zirkonoxid betragen 105 bzw. 200 kN/mm². Besteht das prothetische Material bzw. Implantat aus einem dieser Materialien, werden die Kräfte, die beim Kauen auf die Suprakonstruktion (Prothetik) wirken über den Aufbaupfosten (Abutment) direkt in das Implantat und von diesem auf den Kieferknochen übertragen. Diese starre Struktur entspricht nicht der natürlichen Aufhängung des Zahnes im Kiefer, welche elastisch ist.

Sind die Materialien eines Implantats jedoch zu steif für den Knochen, äußert sich dieses im sogenannten "stress-shielding"-Effekt. Unter "stress-shielding" versteht man eine Verminderung der Knochendichte als Folge des Ausbleibens des normalen Stresses beim Knochen durch ein Implantat, z.B. durch eine femorale Komponente einer Hüftprothese oder ein Kiefer- oder Dentalimplantat. Es ist bekannt, dass sich der Knochen unter normalen Bedingungen aufbaut und an Festigkeit zunimmt, wenn er belastet wird. So passt sich der Knochen in seiner Form an die Funktion an, während der Knochen bei dauerhafter Entlastung, d.h. der Knochen wird nicht oder nur wenig belastet, degeneriert bzw. sich abbaut. Die Veränderung eines Knochens folgt demnach den auf den Knochen wirkenden Kräften und die mechanischen Kräfte bilden die Grundlage für die perfekte Architektur von Knochensubstanz und Knochenstrukturen. Vergleichbare Effekte gelten auch bei Dentalimplantaten.

Sind somit die für ein Implantat, beispielsweise für ein Dentalimplantat, verwendeten Materialien zu steif für den Knochen und fehlt daher die für die Erhaltung und Anpassung der Knochensubstanz erforderliche Stimulierung, büßt der Knochen im Bereich des Implantats aufgrund der Abschirmung der physiologischen Belastungen durch das Implantat Knochenmasse ein und verliert an Dichte und Festigkeit.

Deshalb halten neuerdings sogenannte Hochleistungskunststoffe, wie z.B. Polyetheretherketon (PEEK), Einzug auf diesem Gebiet, da diese ein Elastizitätsmodul (E-Modul) ähnlich dem des knöchernen Gewebes aufweisen, wodurch der Knochen weiterhin die physiologische Belastung erfährt, um nicht an Stabilität einzubüßen. Insofern scheinen diese Hochleistungskunststoffe, insbesondere PEEK, das Material der Wahl zur Herstellung von Implantaten, insbesondere von Dentalimplantaten, darzustellen.

Die US 2007/0111165 A1 beschreibt eine prothetische Dentalvorrichtung mit einem inneren Abschnitt, der aus einem inneren Material ausgebildet ist, und einen äußeren Abschnitt mit einem äußeren Material, das zumindest einen Teil des inneren Abschnitts abdeckt. Das innere Material ist wesentlich verschieden von der Farbe der natürlichen Zähne, während das Außenmaterial im Wesentlichen die gleiche Farbe wie die natürlichen Zähne aufweist. Sowohl das Innenmaterial und das Außenmaterial umfassen ein Polymer. Zur Herstellung werden Press-Formverfahren angegeben. Die Elastizitätsmodule des Innenmaterials und des Außenmaterials unterscheiden sich.

Die FR 2915088 A1 beschreibt ein Implantat, das einen zentralen Kern oder einen Herz (20) aus einem Verbundwerkstoff mit einer thermoplastischen Matrix umfasst, vorzugsweise vom Typ Polyethertherketon (PEEK) verstärkt mit einer Faserverstärkung in Form von kurzen, langen oder kontinuierlichen Fasern, Kohlenstoff, Glas, Aramid oder Metall, und mit einer Hülle, die von einer bio-integrierbaren Polymerbeschichtung (21) gebildet wird, wobei die Hülle am Rand eine oder mehrere flexible Lamellen (22) aufweist, so dass der Durchmesser des Implantats, gemessen an der Oberseite der genannten Lamellen, vor seiner Einführung in eine Bohrung größer ist als der Durchmesser der Bohrung.

Die US 2009/0222091 A1 beschreibt ein Verfahren zur Herstellung einer porösen Struktur aus einem ersten polymeren Material. Das Verfahren umfasst die Handlungen des Mischens des ersten polymeren Materials mit einem zweiten Material (ein wasserlösliches Salz), um eine Mischung zu bilden, wobei das erste Material einen Schmelzpunkt besitzt, der niedriger ist als der des zweiten Materials, das Erhitzen der Mischung unter Druck auf eine Temperatur zwischen dem Schmelzpunkt des ersten Materials und dem Schmelzpunkt des zweiten Materials, Abkühlen der geschmolzenen Mischung bis sie sich verfestigt, und Entfernen des zweiten Materials, also des ein wasserlösliches Salzes (z.B. NaCl) aus dem ersten Material. Das Verfahren kann auch einen nachfolgenden Wärmebehandlungsschritt umfassen. Es wird auch ein geeignetes Material für Implantat, beispielsweise Vertebral- oder Wirbelsäulenimplantate, umfassend ein starres biokompatibles Polymer, wie PEEK, welches eine Vielzahl von verbundenen Poren aufweist. Das Polymer hat zur Veranschaulichung eine Porosität von zwischen 50% und 85% (Volumen) und ist in einer bestimmten Ausführungsform in der Lage, Drücken von bis zu 20 MPa zu widerstehen. Das poröse PEEK-Material kann in jeder Dimension eine minimale Dicke von einem 1 Zoll aufweisen.

Allerdings müssen diese Hochleistungskunststoffe oftmals durch Zusätze verstärkt werden, um bestimmten mechanischen Anforderungen der Praxis standzuhalten. So werden viele Implantate, wie z.B. Osteosyntheseplatten und insbesondere Dentalimplantate, durch Schrauben fixiert, wobei z.B. Dentalimplantate ein spezielles zentrales Innengewinde aufweisen, um einen Aufbaupfosten (Abutment), auf dem wiederum der künstliche Zahnersatz fixiert werden kann, im Dentalimplantat festschrauben zu können. Ein solches Innengewinde muss aus einem Material gefertigt sein, welches sehr hohen mechanischen Beanspruchungen durch die eingebrachte Schraube standhalten kann. Geeignete verstärkte Hochleistungskunststoffe sind faserverstärkt, z.B. faserverstärktes PEEK wie insbesondere kohlefasergefülltes PEEK. Diese faserverstärkten Kunststoffmaterialien, die für ein Implantat, insbesondere ein Dentalimplantat, als Kernmaterial unverzichtbar sind, weisen jedoch zwangsläufig ein E-Modul auf, welches sehr viel größer als das E-Modul von Knochen ist, welches im Bereich von etwa 18 bis 21 kN/mm² liegt. Auch bei diesen verstärkten Kunststoffmaterialien kann daher das Problem des oben beschriebenen stress-shielding-Effekts auftreten.

Es bestand daher die Aufgabe, Implantat-Halbzeuge aus Kunststoff und deren Herstellung anzugeben, die die vorgenannten Nachteile des Standes der Technik nicht aufweisen und sich zur Verwendung als Implantat oder zur Fertigung eines Implantats daraus, insbesondere zur Verwendung als Dentalimplantat oder zur Fertigung eines Dentalimplantats eignen. Diese Implantat-Halbzeuge aus Kunststoff sollen stress-shielding-Effekte möglichst weitgehend vermeiden und genügend hohe mechanische Festigkeit aufweisen, um eine sichere, d.h. z.B. dauerhafte und den hohen mechanischen Beanspruchungen gewachsene, Befestigung weiterer Implantat-Komponenten, z.B. durch Verschrauben, zu gewährleisten.

Die Aufgabe wird gelöst durch das nachfolgend beschriebene erfindungsgemäße Implantat-Halbzeug aus Kunststoff, dessen Herstellung sowie die Verwendung des Implantat-Halbzeug als Implantat oder zur Fertigung eines Implantats daraus, insbesondere die Verwendung als Dentalimplantat oder zur Fertigung eines Dentalimplantats. Die erfindungsgemäße Lösung eignet sich darüber hinaus auch allgemein für die Bereitstellung und Verwendung von Implantat-Halbzeugen als Basis zur Behebung von vielfältigen Knochendefekten bzw. Rekonstruktion von Knochen oder zur Fertigung eines Knochenimplantats zur Behebung von Knochendefekten. Die Erfindung betrifft daher auch solche Knochenimplantate, insbesondere als eine Implantatplatte zur Rekonstruktion von Knochen bzw. zur Behebung von Knochendefekten oder zur Fertigung einer solchen Implantatplatte.

Erfindungsgemäß wurde nun gefunden, dass bei Verwendung von Kunststoffmaterialien mit einem hohen E-Modul, welches, insbesondere sehr viel, größer als das E-Modul von Knochen ist, dennoch ein stress-shielding-Effekt weitestgehend bis sogar ganz vermieden werden kann, wenn diese Kunststoffmaterialien (B) in geeigneter Weise nahtlos mit einem für den Kontakt mit dem biologischen Material, z.B. für den Kontakt mit Knochensubstanz, vorgesehenen, biokompatiblen Kunststoffmaterial (A) mit einem niedrigerem Elastizitätsmodul als das des Kunststoffmaterials (B) ausgerüstet werden. Hierbei ist das Elastizitätsmodul des biokompatiblen Kunststoffmaterials (A) insbesondere dem Elastizitätsmodul von Knochen ähnlich.

In einer ersten Ausgestaltung betrifft die Erfindung daher ein Implantat-Halbzeug aus Kunststoff, welches sich dadurch auszeichnet, dass es einen Verbund aus wenigstens einer, für den Kontakt mit einem biologischen Material vorgesehenen, biokompatiblen (ersten) thermoplastischen Kunststoffeinheit (A) mit einem Elastizitätsmodul E_{A} und wenigstens einer (zweiten) thermoplastischen Kunststoffeinheit (B) mit einem Elastizitätsmodul E_{B} umfasst, wobei für die Elastizitätsmodule dieser Kunststoffeinheiten E_{A} < E_{B} gilt, und die Kunststoffeinheiten in ihrer Verbundzone durch ein thermisches Fügeverfahren nahtlos miteinander verbunden sind, wobei die Kunststoffeinheiten nahtlos so miteinander verbunden sind, dass in der Verbundzone ein Gradient vom Elastizitätsmodul E_{B} der Kunststoffeinheit (Material B) zum Elastizitätsmodul E_{A} der Kunststoffeinheit (Material A) vorliegt und
wobei das Elastizitätsmodul E_{B} der thermoplastischen Kunststoffeinheit (B) 30 bis 110 GPa beträgt.

Als Halbzeug im Sinne der Erfindung werden vorgefertigte Rohmaterialformen für beispielsweise Knochenimplantate, z.B. allgemein für Knochenimplantate zur Rekonstruktion von Knochen bzw. zur Behebung von Knochendefekten und insbesondere für Dentalimplantate, aus Kunststoff verstanden, die jeweils in Material- und Oberflächenqualität, Form und Abmessungen sowie in ihren Toleranzen teilweise fertig erzeugte und in der Regel genormte Vorprodukte (Halbfabrikate oder Halbfertigprodukte) sind, welche in einer späteren Verarbeitung für den bestimmungsgemäßen Verwendungszweck fertig produziert werden können. Die Halbzeuge sind hierbei als Halbfabrikate oder Halbfertigprodukt in der Regel so ausgelegt, dass sie in Form und Abmessungen dem herzustellenden Produkt, z.B. dem Implantat und insbesondere dem Dentalimplantat, möglichst optimal entsprechen.

Die Material- und Oberflächenqualitäten sind zweckmäßigerweise für den bestimmten Einsatzzweck und das Fertigungsverfahren optimiert. Gleiches gilt analog für andere Knochenimplantate.

Elastizität bezeichnet die Eigenschaft eines Körpers oder Werkstoffes, insbesondere bei bestimmungsgemäßen Einsatz, reversibel auf eine einwirkende Kraft zu reagieren. Elastizität im Sinne der Erfindung ist somit die Eigenschaft des Implantat-Halbzeugs bzw. der daraus hergestellten Implantate, insbesondere der Dentalimplantate, unter Krafteinwirkung seine Form zu verändern und bei Wegfall der einwirkenden Kraft in die Ursprungsform zurückzukehren. Beim Implantat-Halbzeug bzw. den daraus hergestellten Implantaten, entspricht die Elastizität im Wesentlichen natürlichen elastischen Bedingungen des Knochens, d.h. insbesondere bei Dentalimplantaten entspricht die Elastizität im Wesentlichen der natürlichen elastischen Aufhängung des Zahnes im Kiefer.

Um die Elastizität größenmäßig für bestimmte Einsatzzwecke von Materialien behandeln zu können, wird die Elastizität von Materialien mit dem Elastizitätsmodul (angegeben in kN/mm²), dem Kompressionsmodul und dem Schubmodul detailliert beschrieben. Diese Module sind über die Poissonzahl miteinander verknüpft. Das Elastizitätsmodul beschreibt hierbei den Zusammenhang zwischen Spannung und Dehnung bei der Verformung eines festen Körpers bei linear elastischem Verhalten. Mit dem Kompressionsmodul wird berechnet, welche allseitige Druckänderung nötig ist, um eine bestimmte reversible Volumenänderung hervorzurufen. Das Schubmodul gibt Auskunft über die lineare elastische Verformung eines Bauteils infolge einer Scherkraft oder Schubspannung.

Im Rahmen der vorliegenden Erfindung ist insbesondere das Elastizitätsmodul, angegeben in kN/mm²) von Bedeutung. Die Erfindung betrifft daher ein Implantat-Halbzeug aus Kunststoff, welches sich dadurch auszeichnet, dass es einen Verbund aus wenigstens einer, für den Kontakt mit einem biologischen Material vorgesehenen, biokompatiblen (ersten) thermoplastischen Kunststoffeinheit (A) mit einem Elastizitätsmodul E_{A} und wenigstens einer (zweiten) thermoplastischen Kunststoffeinheit (B) mit einem Elastizitätsmodul E_{B} umfasst, wobei für die Elastizitätsmodule dieser Kunststoffeinheiten E_{A} < E_{B} gilt, und die Kunststoffeinheiten in ihrer Verbundzone durch ein thermisches Fügeverfahren nahtlos so miteinander thermisch verbunden sind, dass in der Verbundzone ein Gradient vom Elastizitätsmodul E_{B} der Kunststoffeinheit (Material B) zum Elastizitätsmodul E_{A} der Kunststoffeinheit (Material A) vorliegt.

Die Verbbundzone, d.h. der nahtlose Übergang zwischen der thermoplastischen Kunststoffeinheit (A) und einer thermoplastischen Kunststoffeinheit (B) kann ganz oder teilweise glatt und/oder verzahnt gestaltet sein. Die Verbundzone, die z.B. mikroskopisch im Querschnitt untersucht werden kann, ist in der Regel bis zu etwa 2 mm breit, bzw. ragt von der Grenzfläche der Kunststoffeinheiten jeweils bis zu etwa 1 mm in die thermoplastischen Kunststoffeinheit (A) und die thermoplastischen Kunststoffeinheit (B) hinein. Die Verbundzone kann beispielsweise auch nur bis zu etwa 1 mm breit sein, bzw. von der Grenzfläche nur bis zu etwa 0,5 mm jeweils in die thermoplastischen Kunststoffeinheit (A) und die thermoplastischen Kunststoffeinheit (B) hineinragen. Insbesondere kann die Verbundzone auch nur 0,5 mm breit sein, bzw. dann von der Grenzfläche nur bis zu etwa 0,25 mm jeweils in die thermoplastischen Kunststoffeinheit (A) und die thermoplastischen Kunststoffeinheit (B) hineinragen.

Unter Gradient im Sinne der vorliegenden Erfindung wird das Elastizitätsmodul als eine gerichtete physikalische Größe (Elastizitätsmodul-Gradient) verstanden, und beschreibt in der Verbundzone der thermoplastischen Kunststoffeinheit (A) und der thermoplastischen Kunststoffeinheit (B) ein Gefälle der Werte des Elastizitätsmoduls (kN/mm²) vom höheren Elastizitätsmodul E_{B} der thermoplastischen Kunststoffeinheit (B) zum niedrigeren Elastizitätsmodul E_{A} der thermoplastischen Kunststoffeinheit (B).

Eine zweckmäßige Ausführungsform des erfindungsgemäßen Implantat-Halbzeugs zeichnet sich dadurch aus, dass das Elastizitätsmodul E_{A} der thermoplastischen Kunststoffeinheit (A) etwa 1 bis 6 GPa, vorzugsweise etwa 2 bis 5 GPa, besonders bevorzugt etwa 2,5 bis 4,5 GPa, beträgt.

In weiteren zweckmäßigen Ausführungsformen des erfindungsgemäßen Implantat-Halbzeugs zeichnen sich diese dadurch aus, dass das Verhältnis des Elastizitätsmoduls E_{A} der thermoplastischen Kunststoffeinheit (A) zum Elastizitätsmodul E_{B} der thermoplastischen Kunststoffeinheit (B) E_{A} < 1,1 E_{B} ist, und vorzugsweise E_{A} < 1,5 E_{B} ist. In besonders zweckmäßigen Ausführungsformen ist das Verhältnis des Elastizitätsmoduls E_{A} der thermoplastischen Kunststoffeinheit (A) zum Elastizitätsmodul E_{B} der thermoplastischen Kunststoffeinheit (B) E_{A} < 1,1 bis 50 E_{B}, und vorzugsweise E_{A} < 1,5 bis 50 E_{B}. In einer weiteren Ausführungsformen kann das das Verhältnis des Elastizitätsmoduls E_{A} der thermoplastischen Kunststoffeinheit (A) zum Elastizitätsmodul E_{B} der thermoplastischen Kunststoffeinheit (B) E_{A} < 5 bis 44 E_{B} betragen.

In den Ausführungsformen der Erfindung, z.B. im Implantat-Halbzeug oder in einem daraus hergestellten Implantat, kann die für den Kontakt mit einem biologischen Material, d.h. insbesondere für den Kontakt mit Knochen, vorgesehene Kunststoffeinheit (A) auch als außenliegend und die Kunststoffeinheit (B) auch als innenliegend ausgebildet werden. Die Kunststoffeinheit (A) kann dann vorzugsweise eine Hülle oder einen Mantel bilden, wobei die Kunststoffeinheit (B) dann einen Kern oder einen Zylinder bildet, der von dieser Hülle oder diesem Mantel der Kunststoffeinheit (A) umgeben bzw. darin eingelassen ist. Entsprechend kann das Material der Kunststoffeinheit (A) auch als Hüllmaterial oder Mantelmaterial und das Material der Kunststoffeinheit (B) auch als Kernmaterial bezeichnet werden.

In einer ersten Variante dieser Ausgestaltung betrifft die Erfindung daher ein Implantat-Halbzeug aus Kunststoff, welches sich dadurch auszeichnet, dass es einen Verbund aus wenigstens einer, für den Kontakt mit einem biologischen Material vorgesehenen, biokompatiblen, außen liegenden, vorzugsweise als Hülle oder als Mantel ausgebildeten, (ersten) thermoplastischen Kunststoffeinheit (A) mit einem Elastizitätsmodul E_{A} und wenigstens einer innen liegenden, vorzugsweise als Kern ausgebildeten, (zweiten) thermoplastischen Kunststoffeinheit (B) mit einem Elastizitätsmodul E_{B} umfasst, wobei für die Elastizitätsmodule dieser Kunststoffeinheiten E_{A} < E_{B} gilt, und die Kunststoffeinheiten in ihrer Verbundzone durch ein thermisches Fügeverfahren nahtlos miteinander verbunden sind, wobei die Kunststoffeinheiten in ihrer Verbundzone durch ein thermisches Fügeverfahren nahtlos so miteinander verbunden sind, dass in der Verbundzone ein Gradient vom Elastizitätsmodul E_{B} der Kunststoffeinheit (Material B) zum Elastizitätsmodul E_{A} der Kunststoffeinheit (Material A) vorliegt.

Im erfindungsgemäßen Implantat-Halbzeug ist der Kunststoff in einer oder mehrerer der Kunststoffeinheiten, jeweils unabhängig voneinander, ein biokompatibler Kunststoff, vorzugsweise ein biokompatibler thermoplastischer Hochleistungskunststoff.

Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßen Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der Eigenschaften eines Implantatmaterials an das biologische Material, mit dem es in Kontakt steht, mit dem Ziel einer klinisch erwünschten Wechselwirkung. Der Begriff "biokompatibel" bezeichnet somit im Rahmen der Erfindung eine biologische Verträglichkeit und Akzeptanz eines Materials, z.B. eines Kunststoffmaterials für ein erfindungsgemäßes Implantat-Halbzeug, mit lebendem Material, z.B. im Rahmen der Erfindung insbesondere mit Knochengewebe aber auch mit Mucosa oder ggf. anderen mit dem Material in Kontakt tretenden Geweben.

Das erfindungsgemäße Implantat-Halbzeug zeichnet sich in einer Ausführungsform dadurch aus, dass der Kunststoff in einer oder mehreren der Kunststoffeinheiten, jeweils unabhängig voneinander, ausgewählt ist aus der Gruppe PEEK, PA, PE, POM, PMMA, PVAc, PU, PAEK; vorzugsweise PEEK, oder Polymerblends bzw. Kunststoffblends derselben oder auf Basis derselben.

Das erfindungsgemäße Implantat-Halbzeug zeichnet sich in einer Ausführungsform dadurch aus, dass der Kunststoff der thermoplastischen Kunststoffeinheit (A) ausgewählt ist aus der Gruppe PEEK, PA, PE, POM, PMMA, PVAc, PU, PAEK; vorzugsweise PEEK, oder Polymerblends bzw. Kunststoffblends derselben oder auf Basis derselben.

Das erfindungsgemäße Implantat-Halbzeug zeichnet sich in einer Ausführungsform dadurch aus, dass der Kunststoff der thermoplastischen Kunststoffeinheit (B) ausgewählt ist aus der Gruppe PEEK, oder Polymerblends bzw. Kunststoffblends derselben oder auf Basis derselben.

Im erfindungsgemäßen Implantat-Halbzeug kann der Kunststoff in einer oder mehrerer der Kunststoffeinheiten, jeweils unabhängig voneinander, auch ein Polymerblend bzw. Kunststoffblend aus zwei oder mehreren Kunststoffen, insbesondere aus zwei oder mehreren biokompatiblen Kunststoffen, vorzugsweise aus zwei oder mehreren biokompatiblen thermoplastischen Hochleistungskunststoffen, sein. Solche Polymerblends können z.B. aus den vorgenannten Kunststoffen ausgewählt aus der Gruppe PEEK, PA, PE, POM, PMMA, PVAc, PU, PAEK oder auch Polymerblends bzw. Kunststoffblends eines oder mehrerer dieser Kunststoffe mit einem oder mehreren anderen Polymeren bzw. Kunststoffen, insbesondere anderen biokompatiblen Polymeren bzw. Kunststoffen, gebildet sein. Kunststoffblends der thermoplastischen Kunststoffeinheit (A) basieren zweckmäßigerweise auf Kunststoffen ausgewählt aus der Gruppe PEEK, PA, PE, POM, PMMA, PVAc, PU, PAEK. Kunststoffblends der thermoplastischen Kunststoffeinheit (B) basieren zweckmäßigerweise auf Kunststoffen ausgewählt aus der Gruppe PEEK oder andere thermoplastische Kunststoffe wie PA, PE, POM, PMMA, PVAc, PU, PAEK. Vorzugsweise werden solche Kunststoffblends der Kunststoffeinheiten (A) und (B), jeweils unabhängig voneinander, auf Basis von PEEK gebildet.

Im erfindungsgemäßen Implantat-Halbzeug kann der Kunststoff in einer oder mehrerer der Kunststoffeinheiten, jeweils unabhängig voneinander, auch ein "compoundierter" Kunststoff ("Compound"), insbesondere ein "compoundierter" biokompatibler Kunststoff, vorzugsweise ein "compoundierter" biokompatibler thermoplastischer Hochleistungskunststoff, sein.

Unter dem Begriff "compoundiert" bzw. "Compound" (engl. für Verbundstoff, Zusammensetzung, Mischung) versteht man im Rahmen der Erfindung Kunststoffe, denen zusätzliche Grundstoffe wie Füllstoffe, Verstärkungsstoffe oder andere Additive beigemischt worden sind. Durch die Compoundierung werden somit die Kunststoffe mit mindestens einem Grundstoff miteinander zu einer homogenen Mischung verbunden. Ziel der Compoundierung ist es, die Eigenschaften der Kunststoffe durch die zugesetzten Grundstoffe auf einen Anwendungsfall hin zu modifizieren. Insbesondere können im Rahmen der vorliegenden Erfindung solche Grundstoffe zweckmäßig den Kunststoffen zugesetzt werden, die biologisch verträglich, d.h. biokompatibel, sind.

Den Vorgang zum Herstellen eines Compounds nennt man Compoundierung. Die Compoundierung ist somit ein Veredelungsprozess von Kunststoffen durch Beimischung von Zuschlagstoffen (Füllstoffe, Verstärkungsstoffe, Additive usw.) zur gezielten Optimierung der Eigenschaftsprofile von Kunststoffen. Die Compoundierung erfolgt überwiegend in Extrudern (hauptsächlich gleichläufige Doppelschneckenextruder, aber auch gegenläufige Doppelschneckenextruder, sowie durch Planetwalzenextruder und Ko-Kneter) und umfasst z.B. Verfahrensoperationen wie Fördern, Aufschmelzen, Dispergieren, Mischen, Entgasen und Druckaufbau. Im Fall von Thermoplasten kann die Compoundierung z.B. mit Hilfe von Doppelschneckenextrudern erfolgen.

Die Ziele der Compoundierung sind vielfältig und richten sich nach den gewünschten Eigenschaften des späteren Kunststoffteils. Die wichtigsten Ziele der Compoundierung im Rahmen der Erfindung sind beispielsweise:
- Die Veränderung der mechanischen Eigenschaften des Grundpolymers. Hierbei werden über die Zugabe von Verstärkungs- und Füllstoffen, sowie über eine Schlagzähmodifizierung mechanische Kenngrößen wie die Zugfestigkeit, und die Schlagzähigkeit eingestellt. Insbesondere ist im erfindungsgemäßen Zusammenhang die Beeinflussung des Elastizitätsmoduls von Bedeutung.
- Farbeinstellungen: Über die Zugabe von Pigmenten oder sogenannten Masterbatches wird die vom Kunden gewünschte Farbe eingestellt. Ggf. ist zu berücksichtigen, dass bestimmte Farbeinstellungen die mechanischen Eigenschaften beeinflussen können.
- Zugabe von Verarbeitungshilfsstoffen: Diese Gruppe von Stoffen verbessert im Wesentlichen die Verarbeitung der Polymere. Auf diese Weise wird z. B. durch Entformungshilfsmittel die Entformung im Spritzgießprozess vereinfacht.
- Additive, auch Hilfsstoffe oder Zusatzstoffe genannt, sind Stoffe, die Kunststoffen in geringen Mengen zugesetzt werden, um bestimmte Eigenschaften zu erreichen oder zu verbessern oder zu modifizieren. Additive werden eingesetzt, um einen positiven Effekt auf Herstellung, Lagerung, Verarbeitung oder Produkteigenschaften während und nach der Gebrauchsphase zu erreichen. Additive sind in der Regel auf die jeweilige Anwendung hin optimiert.

Die Erfindung betrifft daher auch Implantat-Halbzeuge, die sich dadurch auszeichnen, dass der Kunststoff in einer oder mehreren der Kunststoffeinheiten, jeweils unabhängig voneinander, ein mit Zusatzstoffen versehener Kunststoff, also compoundierter bzw. zusammengesetzter Kunststoff ist. Solche Zusatzstoffe können im Rahmen der Erfindung z.B. ausgewählt sein aus: Verstärkungsstoffe wie Fasern, z.B. Kohlefasern, Glasfasern; Füllstoffe wie z.B. SiO₂, BaSO₄, Tricalciumphosphat, Tetracalciumphosphat und Biogläser (resorbierbare und/oder stabile Calciumphosphatkeramiken wie z.B. GB9, GB14, AP40); Pigmente wie TiO₂.

Die Erfindung betrifft in einer Ausführungsform Implantat-Halbzeuge, die sich insbesondere dadurch auszeichnen, dass der Kunststoff der thermoplastischen Kunststoffeinheit (A) pulverförmige Zusatzstoffe enthält, vorzugsweise pulverförmige Zusatzstoffe ausgewählt aus der Gruppe Hydroxylapatit, Tricalciumphosphat, Tetracalciumphosphat, Bariumsulfat, Titandioxid, Zinkoxid und Biogläser (resorbierbare und/oder stabile Calciumphosphatkeramiken wie z.B. GB9, GB14, AP40).

Die Erfindung betrifft in einer Ausführungsform Implantat-Halbzeuge, die sich insbesondere dadurch auszeichnen, dass der Kunststoff der thermoplastischen Kunststoffeinheit (B) faserverstärkt, z.B. glasfaser- und/oder kohlefaserverstärkt, vorzugsweise kohlefaserverstärkt, ist.

Wie bereits ausgeführt, ist die Compoundierung im erfindungsgemäßen Zusammenhang für die Beeinflussung bzw. zweckentsprechende Einstellung des Elastizitätsmoduls der Kunststoffeinheiten (A) und (B), jeweils unabhängig voneinander, von Bedeutung. Dies soll beispielhaft anhand der nachfolgenden Übersicht dargestellt werden, über verschiedene medizinisch einsetzbare PEEK-Sorten ohne und mit jeweils beigemengten Grundstoffen, wie z.B. Füllstoffen (Pulver) oder Verstärkungsstoffen (Fasern), und dem jeweiligen E-Modul (E1 - E8, wie hier nachfolgend definiert), die als Kunststoffmaterial (A), (B) und ggf. als weitere Kunststoffmaterialien (C, D, usw.) für die Herstellung eines erfindungsgemäßen Implantat-Halbzeugs herangezogen werden können.
- PEEK ungefüllt/roh: Elastizitätsmodul E1 = 1 - 4 GPa;
- PEEK mit Pulver aus der Gruppe Titandioxid, Bariumsulfat, Tricalciumphosphat, Hydroxylapatit: Elastizitätsmodul E2 = 1 - 6 GPa;
- PEEK mit Glasfasern: kurz/zerschnitten mit Elastizitätsmodul E3 = 5 - 20 GPa; endlos/unorientiert mit Elastizitätsmodul E4 = 20 - 80 GPa; endlos/orientiert mit Elastizitätsmodul E5 = 50 - 160 GPa;
- PEEK mit Kohlefasern: kurz/zerschnitten mit Elastizitätsmodul E6 = 5 - 20 GPa; endlos/unorientiert mit Elastizitätsmodul E7 = 20 - 80 GPa; endlos/orientiert mit Elastizitätsmodul E8 = 50 - 160 GPa.

Die Erfindung betrifft auch die Herstellung erfindungsgemäßer Implantat-Halbzeuge durch geeignete thermische Fügeverfahren. Als Fügen bzw. Fügeverfahren bezeichnet man in der Fertigungstechnik Verfahren, die zwei Bauteile dauerhaft miteinander verbinden. Für Kunststoffe kommt dabei vorwiegend im Stand der Technik ein thermisches Fügeverfahren, z.B. das Schweißen oder die Reibverschweißung, und Kleben zum Einsatz.

Um ein erfindungsgemäßes Implantat-Halbzeug aus zwei oder mehreren verschieden thermoplastischen Kunststoffen (A) und (B) herzustellen, eignen sich verschiedene thermische Fügeverfahren, die beim Zusammenbringen der thermoplastischen Kunststoffeinheiten (A) und (B) mit ausreichender Wärmebildung an der Grenzfläche bzw. in der sich bildenden Verbundzone der thermoplastischen Kunststoffeinheiten (A) und (B) einhergehen.

Ein thermisches Fügeverfahren, z.B. Schweißen oder Reibverschweißung, von Kunststoffen setzt ein Vermögen zur Schmelzbildung voraus. Daher kommen für dieses Verfahren nur Thermoplaste in Frage. Die Wärme zum Aufschmelzen des Materials kann mittels einer elektrischen Induktionsheizung (Heizelementschweißen), heißer Druckluft (Warmgasschweißen), Reibung der Moleküle gegeneinander (Hochfrequenzschweißen), Licht- oder Laserstrahlung (Strahlungsschweißen) oder Reibung (Reibungsschweißen) zugeführt werden.

Die angestrebte dauerhafte Verbindung zweier unterschiedlicher Kunststoffteile (A) und (B) kann erfindungsgemäß also durch Einbringen von Energie in Form von Wärme, durch Reibung, durch Wärmebehandlung im Ofen, durch lokale Wärmeapplikation erzeugt durch Heißluft oder LASER oder durch Schmelze bei Umspritzung, erreicht werden. Erfindungsgemäß einsetzbare Verfahren, z.B. Fügeverfahren, können beispielsweise sein: ein Verfahren mittels Aufspritzen oder insbesondere Umspritzen der thermoplastischen Kunststoffeinheit (B) mit der thermoplastischen Kunststoffeinheit (A) im Spritzgussverfahren bzw. durch Extrusion; ein Verfahren mittels Lasersintern der thermoplastischen Kunststoffeinheit (B) mit der thermoplastischen Kunststoffeinheit (A); oder ein Verfahren mittels Reibverschweißung der thermoplastischen Kunststoffeinheit (B) mit der thermoplastischen Kunststoffeinheit (A). Das Verfahren zur Reibverschweißung der thermoplastischen Kunststoffeinheit (B) mit der thermoplastischen Kunststoffeinheit (A) ist besonders bevorzugt.

In einer Ausführungsform betrifft die Erfindung daher ein Verfahren zur Herstellung eines erfindungsgemäßen Implantat-Halbzeugs aus Kunststoff wie vorhergehend beschrieben, welches sich dadurch auszeichnet, dass wenigstens eine, für den Kontakt mit einem biologischen Material vorgesehene, biokompatible (erste) thermoplastische Kunststoffeinheit (A) mit einem Elastizitätsmodul E_{A} mit wenigstens einer (zweiten) thermoplastischen Kunststoffeinheit (B) mit einem Elastizitätsmodul E_{B}, wobei für die Elastizitätsmodule dieser Kunststoffeinheiten E_{A} < E_{B} gilt, durch ein thermisches Fügeverfahren, vorzugsweise durch eine Reibverschweißung, nahtlos miteinander zu einem Verbund verbunden werden.

In einer bevorzugten Ausführungsform betrifft die Erfindung daher ein Verfahren zur Herstellung eines wie vorhergehend beschriebenen bevorzugten erfindungsgemäßen Implantat-Halbzeugs aus Kunststoff, welches sich dadurch auszeichnet, dass die Kunststoffeinheiten (A) und (B) nahtlos so zu einem Verbund miteinander verbunden werden, dass in der Verbundzone ein Gradient vom Elastizitätsmodul E_{B} der (zweiten) Kunststoffeinheit (B) (Material B) zum Elastizitätsmodul E_{A} der (ersten) Kunststoffeinheit (A) (Material A) eingestellt wird.

Die Erfindung betrifft auch die Verwendung der vorhergehend beschriebenen erfindungsgemäßen Implantat-Halbzeuge als Implantat, insbesondere als Knochenimplantat, oder zur Fertigung eines Implantats, vorzugsweise zur Fertigung eines Knochenimplantats. Es kommen hierbei alle Arten von Knochenimplantaten in Frage, beispielweise Implantate für Hüftknochen, Schädelknochen und/oder Kieferknochen. Vorzugsweise finden die erfindungsgemäßen Implantat-Halbzeuge Verwendung als Dentalimplantat oder zur Fertigung eines Dentalimplantats. Es versteht sich für den Fachmann von selbst, dass sämtliche allgemeinen und speziellen Ausführungen zu den oben beschriebenen erfindungsgemäßen Implantat-Halbzeugen gleichermaßen für die erfindungsgemäße Verwendung der Implantat-Halbzeuge zutreffen und angewendet werden können. Die Verwendung der erfindungsgemäßen Implantat-Halbzeuge kann nach den dem Fachmann bekannten einschlägigen Methoden in an sich bekannter Weise erfolgen.

Die Erfindung betrifft weiterhin auch die vorstehend genannten Implantate selbst, insbesondere als Knochenimplantate, die ein hierzu verarbeitetes erfindungsgemäßes Implantat-Halbzeug umfassen. Die erfindungsgemäßen Implantate umfassen hierbei alle Arten von Knochenimplantaten, beispielweise Implantate für Hüftknochen, Schädelknochen und/oder Kieferknochen. Die erfindungsgemäßen Implantate sind vorzugsweise Dentalimplantate. Es versteht sich für den Fachmann von selbst, dass sämtliche allgemeinen und speziellen Ausführungen zu den oben beschriebenen erfindungsgemäßen Implantat-Halbzeugen und deren Verwendung gleichermaßen für die erfindungsgemäßen Implantate und deren Verwendung zutreffen und angewendet werden können. Die Weiterverarbeitung der erfindungsgemäßen Implantat-Halbzeuge zu bzw. die Herstellung von erfindungsgemäßen Implantaten und deren Verwendung kann nach den dem Fachmann bekannten einschlägigen Methoden in an sich bekannter Weise erfolgen.

Die Erfindung wird nachfolgend anhand von Ausführungsbeispielen zu einem Halbzeug als Basis für ein Dentalimplantat sowie als Basis für eine Implantatplatte zur Rekonstruktion von Knochendefekten und anhand der Figuren Fig. 1 bis Fig. 4 näher erläutert werden.
**Fig. 1**: Schematische Darstellung der Verbindung von zwei thermoplastischen Kunststoffen anhand eines Längsschnittes durch das entstandene Implantat-Halbzeug; hier bestehend aus zwei unterschiedlichen PEEK-Compounds (Material A und B); sowie schematische Darstellung der Querschittsebene der Verbundzone von Material A und B.
**Fig. 2****:** Schematische Darstellung eines Implantat-Halbzeuges als Basis für ein Dentalimplantat in Längsansicht und als horizontaler Querschnitt.
**Fig. 3****:** Horizontaler Querschnitt durch den Körper eines zweiteiligen Dentalimplantates.
**Fig. 4**: Schematische Darstellung für ein Implantat-Halbzeug als Basis für die Herstellung einer Platte zur Behebung von Knochendefekten (Knochen-Rekonstruktion), wobei die Materialien A und B wie in den vorangegangenen Darstellungen beschrieben miteinander verbunden sind.

**Abkürzungsverzeichnis**: PA = Polyamid; PE = Polyethylen; PEEK = Polyetheretherketon; PAEK = Polyaryletherketon; PMMA = Polymethylmethacrylat; PU = Polyurethan; POM = Polyoxymethylen; LASER = light amplification by stimulated emission of radiation; E-Modul = Elastizitätsmodul; FE = finite elements; CAD = Computer aided design; CAM = Computer aided manufacturing.

### Knochenimplantate, insbesondere Dentalimplantate

Im Folgenden werden die Kunststoffeinheit (A) auch als Hülle, Mantel oder Hüllmaterial bzw. Mantelmaterial und die Kunststoffeinheit (B) auch als Kern bzw. Kernmaterial bezeichnet.

Materialien wie faserverstärkte Kunststoffe, z.B. kohlefaserverstärktes PEEK, weisen zwar ein E-Modul auf, welches sehr viel größer als das E-Modul von Knochen ist, sind jedoch für auf Kunststoffmaterialien basierende Implantate, insbesondere als Kernmaterial für Dentalimplantate, unverzichtbar. Um zu vermeiden, dass bei diesen verstärkten Kunststoffmaterialien das Problem des oben beschriebenen stress-shielding-Effekts auftritt, wurde mit der vorliegenden Erfindung das Auftragen einer speziellen Mantelschicht auf das Kernmaterial entwickelt, die aus einem Kunststoffmaterial mit einem dem Knochen ähnlichen E-Modul besteht, und so als Pufferzone zwischen hartem Kernmaterial und Knochen fungiert. Gleichzeitig erfüllt diese Mantelschicht, welche eine helle Farbe hat, eine ästhetische Funktion, indem diese das Kernmaterial optisch abschirmt, falls dieses mit Kohlefasern gefüllt ist und deshalb schwarz erscheint, was insbesondere bei Dentalimplantaten bedeutsam ist, da diese in der Mundhöhle teilweise sichtbar sind.

Es kommen biokompatible Materialien zum Einsatz, die ein E-Modul bis 160 KN/mm² und insbesondere kleiner 100 KN/mm² besitzen. Solche Materialien sind insbesondere glas- und kohlenstofffaserverstärkter Kunststoff und Polyetheretherketon (PEEK) und Materialien mit einem höheren E-Modul eignen sich vorzugsweise als Kernmaterial, während Materialien mit einem niedrigeren E-Modul sich vorzugsweise als Hüllmaterial eignen. Diese Materialien weisen E-Module im Bereich von 3,7 KN/mm² (PEEK) und 40 KN/mm² (CFK in Faserrichtung) auf. Sie sind somit wesentlich elastischer als Titan und Zirkonoxid mit einem E-Modul von 105 bzw. 200 KN/mm².

Sowohl im Hinblick auf eine einfache Herstellbarkeit als auch auf eine gewünschte flexible Anpassbarkeit an anatomische Gegebenheiten ist bevorzugt, dass das Implantat einstückig hergestellt ist. Als Werkstoff ist Kunststoff bevorzugt, insbesondere ein Werkstoff aus oder auf Basis von Polyamid oder Polyetherketon, wie vorzugsweise aus Polyetheretherketon (PEEK), oder aus Polyoxymethylen (bspw. Delrin) oder dergleichen. Besonders Polyetheretherketon (PEEK) kann hinsichtlich seines Elastizitätsmoduls sehr gut an das Elastizitätsmodul des Kieferknochens angepasst werden.

Für das Kernelement des Dentalimplantats können grundsätzlich Kunststoffe, wie z.B. PEEK, compoundiert, d.h. eine homogene Mischung aus Zuschlagstoffen und Polymermatrix, hergestellt werden, um durch die eingemischten Zusatzstoffe, z.B. Füllstoffe und/ oder Fasern, unterschiedliche Materialeigenschaften zu erzielen. Bei dieser konventionellen Compoundierung ist das so hergestellte Gesamtmaterial bearbeitet, wobei es zu einer homogenen Durchmischung des Kunststoffes mit den jeweiligen eingemischten Zusatzstoffen kommt. Kommen derartige Materialien z.B. in Form von Implantaten im Kontakt mit menschlichen Knochen zur dauerhaften Anwendung, muss auch der Tatsache Rechnung getragen werden, dass Knochengewebe nicht nur ein niedrigeres E-Modul als die meisten Implantate hat, sondern auch ein Material mit lokal unterschiedlich elastischen Bereichen darstellt. Erfindungsgemäß wird dem entsprochen werden, dass zwei oder mehr thermoplastische Kunststoffe (z.B. PA,PU, PE, PAEK, PEEK) oder zwei oder mehr verschiedene Compounds eines Kunststoffes miteinander verbunden werden, wobei aufgrund der verschiedenen elastischen Eigenschaften, E-Module der einzelnen Komponenten ein neues Implantat-Halbzeug mit lokal veränderbaren und gezielt einstellbaren elastischen Eigenschaften respektive mit einem E-Modul Gradienten, vorwiegend für die medizinischen Anwendung entsteht.

Das Kernelement umfasst hierbei Materialkombinationen aus Kunststoff, vornehmlich PEEK, mit gradierten elastischen Eigenschaften zur Herstellung von medizinisch einsetzbaren Strukturen mit graduierter Steifigkeit im Querschnitt; mit graduierter Elastizität längs, quer, lokal verteilt; und/oder mit variabler Härte an der Oberfläche.

Die angestrebte dauerhafte Verbindung zweier unterschiedlicher Kunststoffteile wird durch Einbringen von Energie in Form von Wärme, durch Reibung, durch Wärmebehandlung im Ofen, durch lokale Wärmeapplikation durch Heißluft oder LASER oder durch Schmelze bei Umspritzung erzeugt. Damit können Dentalimplantate oder auch andere Implantatstrukturen, die dauerhaft im menschlichen Körper verbleiben, gefertigt werden, welche auch ein Schraubengewinde mit Fixationsschraube aufnehmen können.

Durch die Erfindung gelingt eine Optimierung der mechanischen Eigenschaften an den Applikationsort, der farblichen Gestaltung, d.h. der Zahnfarben, der Handhabung und Dauerhaftigkeit sowie der biologischen Kontaktfläche.

Kombinationen können neben reinen PEEK-Kombinationen auch solche aus verschiedenen Kunststoffen wie PMMA und PEEK, PA und PEEK oder HDPE und PEEK sein, soweit die Biokompatibilität gewährleistet ist. Mögliche zur Anwendung kommende PEEK-Varianten sind im obigen Beschreibungsteil aufgelistet und erläutert.

Um ein Halbzeug aus zwei oder mehreren verschieden compoundierten thermoplastischen Kunststoffen herzustellen, eignen sich verschiedene Verfahren wie z.B. Umspritzen im Spritzgussverfahren/Extrusion, Lasersintern, Reibverschweißung.

Im Folgenden soll die Herstellung eines Halbzeuges aus zwei unterschiedlich compoundierten PEEK-Sorten am Beispiel der Reibverschweißung dargestellt werden. Soweit nicht anders angegeben, sind %-Angaben als Gew.-% zu verstehen.

### Herstellung eines Dentalimplantats durch Reibung

Dem Verfahren der Reibverschweißung als Beispiel für die Herstellung eines Halbzeuges aus mindestens zwei unterschiedlichen Thermoplasten, vorzugsweise PEEK-Sorten, liegt das Prinzip zugrunde, dass durch Reibung ein entsprechend hoher Energieeintrag mit einem Maximum an den Grenzflächen der beiden zu verschweißenden Thermoplasten gewährleistet werden kann, welcher oberhalb der jeweiligen Schmelzpunkte der zu verschweißenden Materialien liegt und dazu führt, dass die jeweils gegeneinander geriebenen Oberflächen angeschmolzen werden, was dann zu einer festen Verschweißung der beiden thermoplastischen Kunststoffe führt.

Im vorliegenden Fall wird die zur Verschweißung notwendige Wärmeenergie mithilfe von Rotationsbewegungen um die Achse eines rotationssymmetrischen Kunststoffteils (B) (Materials B) unter stetigem Druck des Kunststoffteils (B) (Materials B) gegen ein Kunststoffteil (A) (Material A) erzeugt, wobei Material B das Kernmaterial und Material A das Mantelmaterial darstellt. Dazu wird Material B in eine zuvor durch Bohrung erzeugte zylinderförmige Hohlform im Material A unter Druck eingebracht, wobei die Bohrung im Durchmesser um 0,1-10%, bevorzugt 5% geringer ist als der Durchmesser des im Querschnitt runden Materials B, um eine primäre Presspassung zwischen den beiden Materialien zu gewährleisten.

Anschließend wird unter einem steten vertikalen Druck von 10 - 100 N, vorzugsweise 50 N, der über die Längsachse des Materials B gegen A einwirkt, das Material B innerhalb der im Material A befindlichen Bohrung mit einer Umdrehungszahl von 200 - 1500 U/min, hier vorzugsweise 300 U/min, für 0,5 - 5 Sekunden, hier vorzugsweise 1 Sekunde, um seine Längsachse gedreht. Die dabei entstandene Verschweißung der beiden Materialien muss nach Abschalten der Rotationsbewegungen unter Aufrechterhaltung des vertikalen Drucks bei Raumtemperatur abkühlen, vorzugsweise über einen Zeitraum von mindestens 20 Minuten.

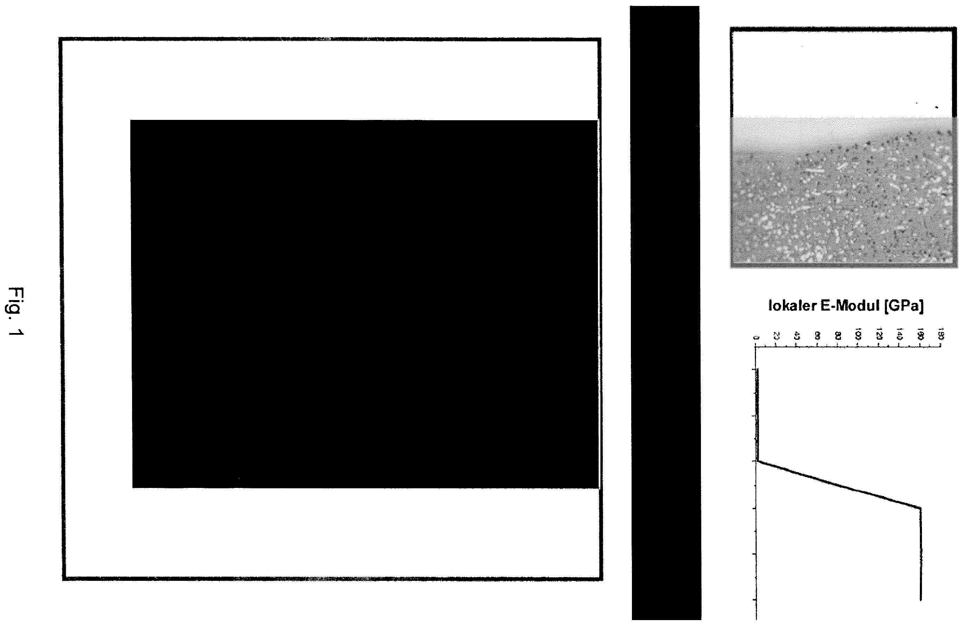

In Fig.1 ist das Ergebnis dieses Verfahrens beispielhaft schematisch dargestellt, wobei hier ein mit 10% titandioxidgefülltes PEEK als Material A, und ein 30% kohlefasergefülltes PEEK als Material B herangezogen worden waren. Die verwendeten Materialien sind wie folgt weiter charakterisiert:
- Material A: z.B. pulvergefülltes PEEK mit einem E-Modul E2 = 1-6 GPa; hier mit TiO₂-geffüllt; PEEK mit E-Modul ≥ 3 GPa.
- Material B: z.B. fasergefülltes PEEK mit einem E-Modul E8 = 50-160 GPa; hier zu 30% mit kurzen Kohlefasern gefülltes PEEK mit E-Modul ≥ 20 GPa.

Im Mikroskopbild der Querschnittebene durch die Verbindung von A+B beobachtet man einen nahtlosen Übergang zwischen Material A und B, wobei die Wände der Materialien glatt und/oder verzahnt gestaltet sein können. Graphisch lässt sich dieses durch einen E-Modulgradienten innerhalb einer Schnittlinie durch den Bereich der festen Verbindung von Material A und B verdeutlichen.

Grundsätzlich basieren Material A und B auf einem thermoplastischen Kunststoff, vorzugsweise PEEK, welche jeweils homogen mit unterschiedlichen Zusatzstoffen gefüllt oder ungefüllt sein können, woraus sich für A und B jeweils unterschiedliche mechanische Eigenschaften und somit unterschiedliche E-Module, wie oben definiert als E1- E8, ergeben; hierbei können sich folgende Verhältnisse von E-Modul Material A zu E-Modul Material B unter Verwendung von zwei Materialien mit einem jeweiligen E-Modul aus dem Bereich E1-E8 ergeben: E-Material B>1,1xE-Material A, bevorzugt im Verhältnis E-Material B >1,5-50xE-Material A; im Verlauf durch die nahtlos miteinander verbundenen Kunststoffeinheiten treten dadurch unterschiedliche Elastizitätsgradienten auf, wodurch das daraus resultierende Halbzeug andere Materialeigenschaften sowohl gegenüber A alleine als auch gegenüber B alleine erhält (siehe Fig.1). Grundsätzlich können bei Bedarf auch mehr als zwei Materialien auf diese Weise miteinander dauerhaft verbunden werden, insofern wäre ein drittes Grundmaterial als Material C und ein viertes Grundmaterial als Material D usw. zu definieren.

### Anwendung in der Implantoloaie

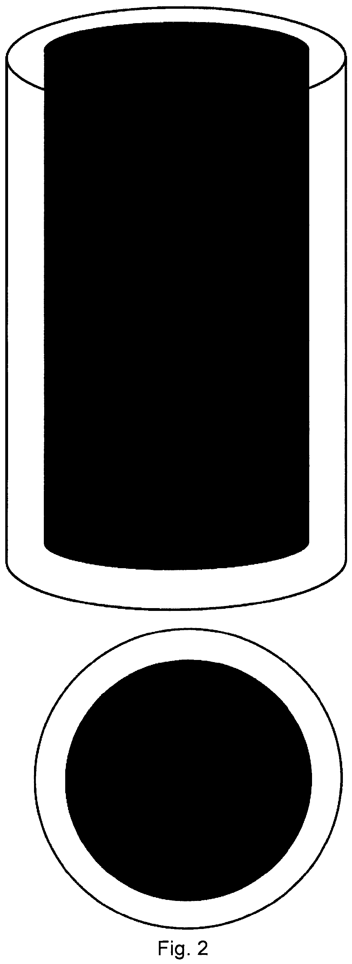

In Fig.2 ist ein auf die vorstehend beschriebene Weise hergestelltes Implantat-Halbzeug dargestellt, welches speziell zur Herstellung eines Dentalimplantatkörpers dient, welcher beispielsweise mit CAD/CAM-Technik weiter zum fertigen Medizinprodukt umgearbeitet werden kann.

So zeigt die Fig.2 in schematischer Darstellung (Längsansicht/Horizontaler Querschnitt) ein Implantat-Halbzeug als Basis für ein Dentalimplantat mit einem Durchmesser von Material B ≥ 2mm, vorzugsweise mit einem E-Modul im Bereich E3 - E8; mit einem nahtlosen Übergang zwischen Material A und Material B, wobei die Wände der Materialien in der Verbundzone glatt oder verzahnt gestaltet sein können.

Die Umarbeitung des für die Herstellung eines Dentalimplantates dienenden Implantat-Halbzeugs beinhaltet insbesondere das Einbringen eines zentralen Hohlraumes, welcher gegebenenfalls mit einem Innengewinde ausgekleidet ist, um das fixieren eines Aufbaupfostens (Abutment), ggf. mit einer Schraube, zu gewährleisten.

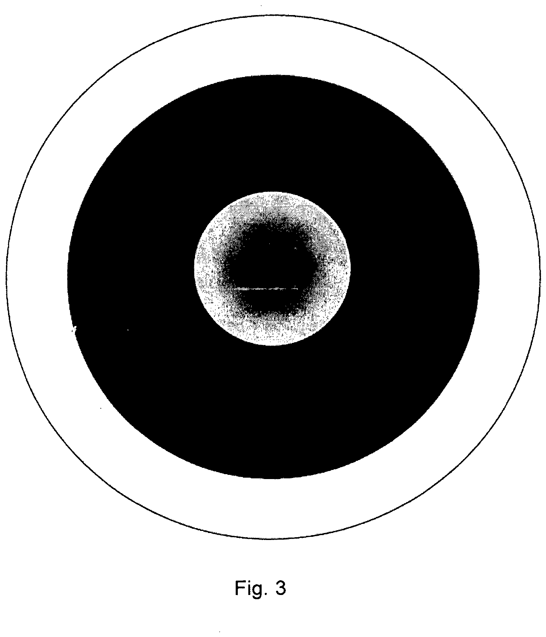

In Fig. 3 ist der horizontale Querschnitt durch ein solches Dentalimplantat schematisch dargestellt. Fig. 3 zeigt dabei einen nahtlosen Übergang zwischen Material A und Material B, wobei die Wände der Materialien in der Verbundzone wiederum glatt oder verzahnt gestaltet sein können. Die Schichtdicke von Material A beträgt ≥ 0,2mm, bevorzugt 0,5-1,5 mm, und vorzugsweise liegt ein Material A mit einem E-Modul im Bereich E1 - E2 vor. Der Durchmesser von Material B beträgt ≥ 2mm, und vorzugsweise liegt ein Material B mit einem E-Modul im Bereich E3 - E8 vor. Fig. 3 zeigt weiterhin einen zentralen Hohlraum in Richtung der Längsachse, welcher zur Verankerung eines Sekundärteiles/Abutments dient; im Falle einer Verankerung durch eine zentrale Schraube befindet sich in dem Hohlraum ein Innengewinde zum Befestigen der Schraube; der Durchmesser des zentralen Hohlraumes beträgt ≥ 0,9 mm, belässt aber noch eine genügende Wandstärke des Materials B.

### Zusammenfassung der Ergebnisse zu erfindungsgemäßen Implantat-Halbzeugen und der Verwendung bzw. Weiterverarbeitung

a) Ziele, die mit der vorliegenden Erfindung erreicht werden:
   - durch Materialkombination wird ein E-Modulgradient von innen nach außen geschaffen;
   - durch stabiles Kernmaterial werden mechanisch belastbare Verbindungsmöglichkeiten v.a. durch Schrauben (Abutmentbefestigung) ermöglicht;
   - durch ein E-Modul ähnlich dem umgebenden Knochen im Randbereich gelingt die Verhinderung des Stress-Shieldings;
   - durch Compoundierung des Außenmaterials bzw. Hüllenmaterials kann die Knochenintegration sichergestellt und optimiert werden;
   - durch Compoundierung des Außenmaterials bzw. Hüllenmaterials kann eine farbliche Gestaltung für bessere Ästhetik erreicht werden;
   - durch Compoundierung gelingt die Herstellung der Röntgenopazität;
   - durch 3-D-Modellberechnung gelingt eine optimierte materialbezogene Implantatgeometrie;
b) Anforderungen an die Kombination der Materialien A (Hülle) und B (Kern):
   1. E-Modul Material A < E-Modul Material B;
   2. homogene Verbindung entsprechend einer Verschweißung;
   3. heterogene Verbindung durch Verklebung, Einsatz von Haftvermittlern / Bondern / Adhäsiven, Oberflächenvorbehandlung;
   4. farbliche Gestaltung, wobei A immer heller (weiß bis zahnfarben)als B ist;
   5. Bearbeitbarkeit durch thermoplastische Formgebung und/oder Spanabtrag;
   6. Materialkombinationen:
      Innen (Kern) = Material B: PEEK oder andere faserverstärkte thermoplastische Kunststoffe (vornehmlich Kohlefasern);
      Außen (Hülle, Mantel) = Material A: thermoplastischer spritzfähiger Kunststoff mit hohem E-Modul, nachgewiesener Biokompatibilität und gefüllt oder ungefüllt mit Pulver (Hydroxylapatit, Trikalziumphosphat, Bariumsulfat, Titandioxid, Zinkoxid, Biogläser), wie zum Beispiel: PA, PE, POM, PMMA, PVAc, PU, PEEK, PAEK.
c) Mögliche Applikationen:

### 1. Dentalimplantat

a) aus homogenem Material
b) aus E-Modul-gradiertem Material

Im Allgemeinen weist ein zweiteiliges Dentalimplantat ein Außengewinde auf, welches das Eindrehen in das entsprechende Implantatlager im Knochen ermöglicht. In dessen Zentrum ist meist ein Innengewinde eingebracht, welches nach oben hin offen ist, um das zweite Teil, den Aufbaupfosten (Abutment), mit einer zentralen Schraube am Implantat zu befestigen, woran wiederum der künstliche Zahnersatz fixiert wird. Der Verbindungsbereich, in dem sich Implantat und Abutment berühren, kann unterschiedlich gestaltet sein. Vorzugsweise sollte hier eine konische Verbindung herangezogen werden. Das Abutment hat dabei einen basalen Konus, der in das Implantat eingefügt wird, mit einem Gesamtkonuswinkel von 12°. Die Geometrie eines Dentalimplantatkörpers kann unterschiedlich dimensioniert sein, wobei der Außendurchmesser zwischen 3,2 bis 6 mm und die Gesamtlänge zwischen 6 bis 18 mm betragen kann. Getestet wurde ein Implantat mit 4 mm Durchmesser und 8 mm Länge in den 3 Materialvarianten:
- Implantat Nr.1: homogen aus PEEK mit etwa 10% TiO₂-Füllung (entsprechend Material A); Farbe: weiß;
- Implantat Nr.2: homogen aus PEEK verstärkt mit etwa 60% parallelen Endlos-Kohlefasern (entsprechend Material B); Farbe: schwarz;
- Implantat Nr.3: E-Modul-gradiert (Kern: 3 mm Durchmesser, PEEK verstärkt mit etwa 60% parallelen Kohlefasern; Hülle: 0,5 mm Schichtdicke, PEEK mit etwa 10% TiO₂-gefüllt); Farbe: weißer Mantel, schwarzer Kern.

Die Implantate wurden in einer Universalprüfmaschine in einem statischen Druckversuch getestet. Anhand der Ergebnisse aus diesem Druckversuch wurde die Druckfestigkeit ermittelt. Diese betrug für
- Implantat Nr.1 (weiß): 140,1 ± 1,3 MPa
- Implantat Nr.2 (schwarz): 712,7 ± 66 MPa
- Implantat Nr. 3 (weißer Mantel, schwarzer Kern): 610 ± 24 MPa

### 2. Osteosyntheseplatte, bzw. Knochendefektüberbrückungsplatte für Mund-, Kiefer-, Gesichtschirurgie

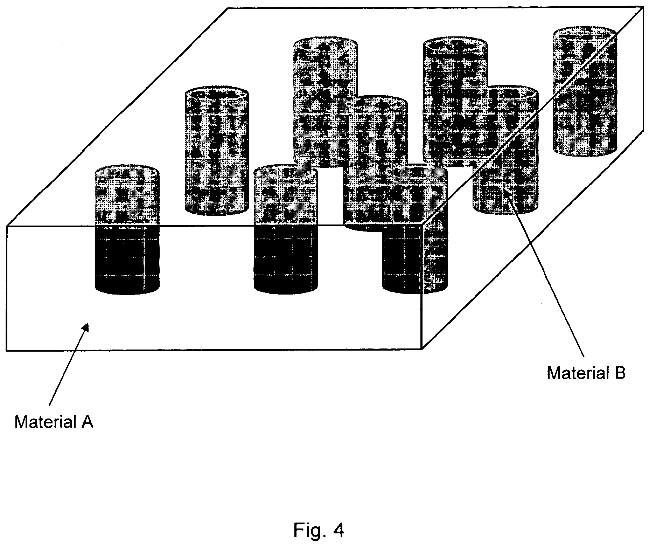

Durch das Herstellungsverfahren der Verschweißung durch Reibung (Reibverschweißung) können beispielsweise auch mehrere Elemente des Materials B in eine Grundform des Materials A dauerhaft eingebracht werden, um dadurch ein stabiles Widerlager für Schrauben zu bilden. Ein derartiges Implantat-Halbzeug ist in Fig. 4 aufgeführt, welches beispielsweise zur Rekonstruktion von Defekten des knöchernen Schädels dienen kann.

Fig. 4 zeigt hierbei eine schematische Darstellung für ein Implantat-Halbzeug als Basis für die Herstellung einer Platte zur Behebung von Knochendefekten (Knochen-Rekonstruktion), wobei die Materialien A und B wie in den vorangegangenen Darstellungen beschrieben, nahtlos miteinander verbunden sind. Das Material A ist z.B. PEEK mit einem Elastizitätsmodul E1 bzw. E2; das Material B ist z.B. PEEK mit einem Elastizitätsmodul E3 - E8.

## Patentansprüche

1. Implantat-Halbzeug aus Kunststoff, **dadurch gekennzeichnet, dass** es einen Verbund aus wenigstens einer, für den Kontakt mit einem biologischen Material vorgesehenen, biokompatiblen thermoplastischen Kunststoffeinheit (A) mit einem Elastizitätsmodul E_{A} und wenigstens einer thermoplastischen Kunststoffeinheit (B) mit einem Elastizitätsmodul E_{B} umfasst, wobei für die Elastizitätsmodule dieser Kunststoffeinheiten E_{A} < E_{B} gilt, und die Kunststoffeinheiten in ihrer Verbundzone durch ein thermisches Fügeverfahren nahtlos miteinander verbunden sind, wobei die Kunststoffeinheiten nahtlos so miteinander verbunden sind, dass in der Verbundzone ein Gradient vom Elastizitätsmodul E_{B} der Kunststoffeinheit (Material B) zum Elastizitätsmodul E_{A} der Kunststoffeinheit (Material A) vorliegt, und
**dadurch gekennzeichnet, dass** das Elastizitätsmodul E_{B} der thermoplastischen Kunststoffeinheit (B) 30 bis 110 GPa beträgt.

2. Implantat-Halbzeug nach Anspruch 1, **dadurch gekennzeichnet, dass** das Elastizitätsmodul E_{A} der thermoplastischen Kunststoffeinheit (A) 1 bis 6 GPa, vorzugsweise 2 bis 5 GPa, besonders bevorzugt 2,5 bis 4,5 GPa, beträgt.

3. Implantat-Halbzeug nach einem der Ansprüche 1 oder 2, **dadurch gekennzeichnet, dass** das Verhältnis des Elastizitätsmoduls E_{A} der thermoplastischen Kunststoffeinheit (A) zum Elastizitätsmodul E_{B} der thermoplastischen Kunststoffeinheit (B) E_{A} < 1,1 E_{B} ist, und vorzugsweise E_{A} < 1,5 E_{B} ist.

4. Implantat-Halbzeug nach Anspruch 3, **dadurch gekennzeichnet, dass** das Verhältnis des Elastizitätsmoduls E_{A} der thermoplastischen Kunststoffeinheit (A) zum Elastizitätsmoduls E_{B} der thermoplastischen Kunststoffeinheit (B) E_{A} < 1,1 bis 50 E_{B} ist, und vorzugsweise E_{A} < 1,5 bis 50 E_{B} ist.

5. Implantat-Halbzeug nach einem der Ansprüche 1 bis 4, **dadurch gekennzeichnet, dass** der Kunststoff in einer oder mehreren der Kunststoffeinheiten, jeweils unabhängig voneinander, ausgewählt ist aus der Gruppe PEEK, PA, PE, POM, PMMA, PVAc, PU, PAEK; vorzugsweise PEEK oder Polymerblends derselben oder auf Basis derselben.

6. Implantat-Halbzeug nach einem der Ansprüche 1 bis 5, **dadurch gekennzeichnet, dass**
i) der Kunststoff der thermoplastischen Kunststoffeinheit (A) ausgewählt ist aus der Gruppe PEEK, PA, PE, POM, PMMA, PVAc, PU, PAEK; vorzugsweise PEEK
und/oder
ii) der Kunststoff der thermoplastischen Kunststoffeinheit (B) ausgewählt ist aus der Gruppe PEEK oder PAEK.

7. Implantat-Halbzeug nach einem der Ansprüche 1 bis 6, **dadurch gekennzeichnet, dass** der Kunststoff in einer oder mehreren der Kunststoffeinheiten, jeweils unabhängig voneinander, ein mit Zusatzstoffen versehener Kunststoff (compoundierter/zusammengesetzter Kunststoff) ist, vorzugsweise mit Zusatzstoffen ausgewählt aus der Gruppe der Verstärkungsstoffe wie insbesondere Fasern, vorzugsweise Kohlefasern oder Glasfasern, besonders bevorzugt Kohlefasern; Füllstoffe, insbesondere SiO₂, BaSO₄, Tricalciumphosphat, Tetracalciumphosphat und Biogläser; Pigmente, insbesondere TiO₂.

8. Implantat-Halbzeug nach einem der Ansprüche 1 bis 7, **dadurch gekennzeichnet, dass** der Kunststoff der thermoplastischen Kunststoffeinheit (A) pulverförmige Zusatzstoffe enthält, vorzugsweise pulverförmige Zusatzstoffe ausgewählt aus der Gruppe Hydroxylapatit, Tricalciumphosphat, Bariumsulfat, Titandioxid, Zinkoxid und Biogläser.

9. Implantat-Halbzeug nach einem der Ansprüche 1 bis 8, **dadurch gekennzeichnet, dass** der Kunststoff der thermoplastischen Kunststoffeinheit (B) faserverstärkt, vorzugsweise kohlefaserverstärkt, ist.

10. Implantat-Halbzeug nach einem der Ansprüche 1 bis 9, **dadurch gekennzeichnet, dass** die Kunststoffeinheit (A) als außenliegend und die Kunststoffeinheit (B) als innenliegend ausgebildet sind, vorzugsweise so, dass die Kunststoffeinheit (A) eine Hülle oder einen Mantel bildet, wobei die Kunststoffeinheit (B) dann einen Kern oder einen Zylinder bildet, der von dieser Hülle oder diesem Mantel der Kunststoffeinheit (A) umgeben bzw. darin eingelassen ist.

11. Verwendung des Implantat-Halbzeugs nach einem der Ansprüche 1 bis 10 zur Fertigung eines Implantats, vorzugsweise zur Fertigung eines Dentalimplantats.

12. Implantat-Halbzeug nach einem der Ansprüche 1 bis 10 zur Verwendung als Implantat, vorzugsweise als Dentalimplantat.

13. Implantat, vorzugsweise ein Dentalimplantat, umfassend ein hierzu verarbeitetes Implantat-Halbzeug nach einem der Ansprüche 1 bis 10.

14. Verfahren zur Herstellung eines Implantat-Halbzeug aus Kunststoff nach einem der Ansprüche 1 bis 10, **dadurch gekennzeichnet, dass** wenigstens eine, für den Kontakt mit einem biologischen Material vorgesehene, biokompatible thermoplastische Kunststoffeinheit (A) mit einem Elastizitätsmodul E_{A} mit wenigstens einer thermoplastischen Kunststoffeinheit (B) mit einem Elastizitätsmodul E_{B}, wobei für die Elastizitätsmodule dieser Kunststoffeinheiten E_{A} < E_{B} gilt, durch ein thermisches Fügeverfahren, vorzugsweise durch eine Reibverschweißung, nahtlos miteinander zu einem Verbund verbunden werden.

## Claims

1. Implant preform made of plastic, **characterized in that** it comprises an assembly of at least one biocompatible - being intended for contact with a biological material - thermoplastic plastics unit (A) having an elastic modulus E_{A} and at least one thermoplastic plastics unit (B) having an elasticity modulus E_{B}, where for the elasticity moduli of these plastics units E_{A} < E_{B}, and the plastics units in their assembly zone are seamlessly connected to one another by a thermal joining technique, the plastics units being seamlessly connected to one another in such a way that in the assembly zone there is a gradient from the elasticity modulus E_{B} of the plastics unit (material B) to the elasticity modulus E_{A} of the plastics unit (material A), and **characterized in that** the elasticity modulus E_{B} of the thermoplastic plastics unit (B) is 30 to 110 GPa.

2. Implant preform according to Claim 1, **characterized in that** the elasticity modulus E_{A} of the thermoplastic plastics unit (A) is 1 to 6 GPa, preferably 2 to 5 GPa, more preferably 2.5 to 4.5 GPa.

3. Implant preform according to either of Claims 1 or 2, **characterized in that** the ratio of the elasticity modulus E_{A} of the thermoplastic plastics unit (A) to the elasticity modulus E_{B} of the thermoplastic plastics unit (B) is E_{A} < 1.1 E_{B}, and preferably E_{A} < 1.5 E_{B}.

4. Implant preform according to Claim 3, **characterized in that** the ratio of the elasticity modulus E_{A} of the thermoplastic plastics units (A) to the elasticity modulus E_{B} of the thermoplastic plastics unit (B) is E_{A} < 1.1 to 50 E_{B}, and preferably E_{A} < 1.5 to 50 E_{B}.

5. Implant preform according to any of Claims 1 to 4, **characterized in that** the plastic in one or more of the plastics units, in each case independently of one another, is selected from the group of PEEK, PA, PE, POM, PMMA, PVAc, PU, PAEK; preferably PEEK or polymer blends thereof or based thereon.

6. Implant preform according to any of Claims 1 to 5, **characterized in that**
i) the plastic of the thermoplastic plastics unit (A) is selected from the group of PEEK, PA, PE, POM, PMMA, PVAc, PU, PAEK; preferably PEEK
and/or
ii) the plastic of the thermoplastic plastics unit (B) is selected from the group of PEEK or PAEK.

7. Implant preform according to any of Claims 1 to 6, **characterized in that** the plastic in one or more of the plastics units, in each case independently of one another, is an additized plastic (compounded/- composite plastic), preferably with additives selected from the group of the reinforcing agents such as, in particular, fibres, preferably carbon fibres or glass fibres, more preferably carbon fibres; fillers, in particular SiO₂, BaSO₄, tricalcium phosphate, tetracalcium phosphate, and bioglasses; pigments, in particular TiO₂.

8. Implant preform according to any of Claims 1 to 7, **characterized in that** the plastic of the thermoplastic plastics unit (A) comprises additives in powder form, preferably additives in powder form selected from the group of hydroxylapatite, tricalcium phosphate, barium sulphate, titanium dioxide, zinc oxide, and bioglasses.

9. Implant preform according to any of Claims 1 to 8, **characterized in that** the plastic of the thermoplastic plastics unit (B) is fibre-reinforced, preferably carbon fibre-reinforced.

10. Implant preform according to any of Claims 1 to 9, **characterized in that** the plastics unit (A) is designed as external and the plastics unit (B) is designed as internal, preferably such that the plastics unit (A) forms a shell or a jacket, with the plastics unit (B) then forming a core or a cylinder which is surrounded by or inlaid into this shell or this jacket of the plastics unit (A).

11. Use of the implant preform according to any of Claims 1 to 10 for fabrication of an implant, preferably for fabrication of a dental implant.

12. Implant preform according to any of Claims 1 to 10 for use as an implant, preferably as a dental implant.

13. Implant, preferably a dental implant, comprising an implant preform processed thereto and according to any of Claims 1 to 10.

14. Method for producing an implant preform made of plastic, according to any of Claims 1 to 10, **characterized in that** at least one biocompatible - being intended for contact with a biological material - thermoplastic plastics unit (A) having an elasticity modulus E_{A} and at least one thermoplastic plastics unit (B) having an elasticity modulus E_{B}, where for the elasticity moduli of these plastics units E_{A} < E_{B}, are joined seamlessly to one another to form an assembly by a thermal joining technique, preferably by friction welding.

## Revendications

1. Demi-produits d'implant en matière plastique, **caractérisé en ce qu'**il comprend un composite d'au moins une unité (A) de matière plastique thermoplastique biocompatible, prévue le contact avec une matière biologique et ayant un module E_{A} d'élasticité et d'au moins une unité (B) de matière plastique thermoplastique ayant un module E_{B} d'élasticité, dans lequel, pour les modules d'élasticité de ces unités de matière plastique, on a E_{A} < E_{B} et les unités de matière plastique sont, dans leur zone de liaison, reliées l'une à l'autre sans joint par un procédé de jonction thermique, les unités de matière plastique étant reliées l'une à l'autre sans joint, de manière à avoir, dans la zone de liaison, un gradient du module E_{B} d'élasticité de l'unité de matière plastique (matériau B) au module E_{A} d'élasticité de l'unité de matière plastique (matériau A), et **caractérisé en ce que** le module E_{B} d'élasticité de l'unité (B) de matière plastique thermoplastique va de 30 à 110 GPa.

2. Demi-produits d'implant suivant la revendication 1, **caractérisé en ce que** le module E_{A} d'élasticité de l'unité (A) de matière plastique thermoplastique va de 1 à 6 GPa, de préférence de 2 à 5 GPa, d'une manière particulièrement préférée de 2,5 à 4,5 GPa.

3. Demi-produits d'implant suivant l'une des revendications 1 ou 2, **caractérisé en ce que** le rapport du module E_{A} d'élasticité de l'unité (A) de matière plastique thermoplastique au module E_{B} d'élasticité de l'unité (B) de matière plastique thermoplastique est E_{A} < 1,1 E_{B}, et de préférence E_{A} < 1,5 E_{B}.

4. Demi-produits d'implant suivant la revendications 3, **caractérisé en ce que** le rapport du module E_{A} d'élasticité de l'unité (A) de matière plastique thermoplastique au module E_{B} d'élasticité et de l'unité (B) de matière plastique thermoplastique est E_{A} < 1,1 à 50 E_{B}, et de préférence E_{A} < 1,5 à 50 E_{B}.

5. Demi-produits d'implant suivant l'une des revendications précédentes, **caractérisé en ce que** la matière plastique est choisie dans une ou plusieurs des unités de matière plastique, respectivement, indépendamment les une des autres, du groupe PEEK, PA, PE, POM, PMMA, PVAc, PU, PAEK ; de préférence PEEK ou un mélange de polymères de celui-ci ou à base de celui-ci.

6. Demi-produits d'implant suivant l'une des revendications 1 à 5, **caractérisé en ce que**
i) la matière plastique de l'unité (A) de matière plastique thermoplastique est choisie dans le groupe PEEK, PA, PE, POM, PMMA, PVAc, PU, PAEK ; de préférence PEEK et/ou
ii) la matière plastique de l'unité (B) de matière plastique thermoplastique est choisie dans le groupe PEEK ou PAEK.

7. Demi-produits d'implant suivant l'une des revendications 1 à 6, **caractérisé en ce que** la matière plastique, dans l'une ou dans plusieurs des unités de matière plastique, est, respectivement, indépendamment l'une de l'autre, une matière plastique pourvue d'additifs (matière plastique compoundée/composée), en étant choisie de préférence avec des additifs du groupe des substances de renfort, comme notamment des fibres, de préférence des fibres de carbone ou des fibres de verre, d'une manière particulièrement préférée des fibres de carbone ; des charges, notamment SiO₂, BaSO₄, le phosphate de tricalcium, le phosphate de tétracalcium et des bioverres ; des pigments, notamment TiO₂.

8. Demi-produits d'implant suivant l'une des revendications 1 à 7, **caractérisé en ce que** la matière plastique de l'unité (A) de matière plastique thermoplastique contient des additifs pulvérulents, de préférence des additifs pulvérulents choisis dans le groupe de l'hydroxylapatite, du phosphate de tricalcium, du sulfate de baryum, du dioxyde de titane, de l'oxyde de zinc et des bioverres.

9. Demi-produits d'implant suivant l'une des revendications 1 à 8, **caractérisé en ce que** la matière plastique de l'unité (B) de matière plastique thermoplastique est renforcée par de la fibre, en étant renforcée de préférence par de la fibre de carbone.

10. Demi-produits d'implant suivant l'une des revendications 1 à 9, **caractérisé en ce que** l'unité (A) de matière plastique est constituée comme se trouvant à l'extérieur et l'unité (B) de matière plastique comme se trouvant à l'intérieur, de préférence de manière à ce que l'unité (A) de matière plastique forme une gaine ou une enveloppe, l'unité (B) de matière plastique formant alors un noyau ou un cylindre ou étant entourée de la gaine ou de cette enveloppe de l'unité (A) de matière plastique ou y étant insérée.

11. Utilisation du demi-produit d'implant suivant l'une des revendications 1 à 10, pour la fabrication d'un implant, de préférence pour la fabrication d'un implant dentaire.

12. Demi-produit d'implant suivant l'une des revendications 1 à 10, à utiliser comme implant, de préférence comme implant dentaire.

13. Implant, de préférence implant dentaire, comprenant un demi-produit d'implant, traité à cet effet, suivant l'une des revendications 1 à 10.

14. Procédé de fabrication d'un demi-produit d'implant en matière plastique suivant l'une des revendications 1 à 10, **caractérisé en ce que** l'on relie entre elles, sans joint, par un procédé de jonction thermique, de préférence par un procédé de soudure avec friction, au moins une unité (A) de matière plastique thermoplastique biocompatible, prévue pour le contact avec une matière biologique et ayant un module E_{A} d'élasticité, à au moins une unité (B) de matière plastique thermoplastique ayant un module E_{B} d'élasticité, dans lequel, pour les modules d'élasticité de ces unités de matière plastique, on a E_{A} < E_{B}.
